Europäisches Patentamt

European Patent Office

Office européen des brevets

(19)

(11) Veröffentlichungsnummer: **0 381 123**
**A1**

(12) # EUROPÄISCHE PATENTANMELDUNG

(21) Anmeldenummer: **90101753.3**

(22) Anmeldetag: **29.01.90**

(51) Int. Cl.⁵: **G01D 5/42, G01L 7/04**

(30) Priorität: **30.01.89 DE 8901009 U**

(43) Veröffentlichungstag der Anmeldung:
**08.08.90 Patentblatt 90/32**

(84) Benannte Vertragsstaaten:
**AT BE DE DK ES FR GB IT NL SE**

(71) Anmelder: **Baker, Susan**
**41 Rusham Park Avenue**
**Egham, Surrey(GB)**

(72) Erfinder: **Baker, Susan**
**41 Rusham Park Avenue**
**Egham, Surrey(GB)**

(74) Vertreter: **Patentanwälte Dr. Solf & Zapf**
**Zeppelinstrasse 53**
**D-8000 München 80(DE)**

(54) **Anzeigevorrichtung zur Anzeige eines in einem Hohlraum vorhandenen Druckes.**

(57) Bei einer Anzeigevorrichtung zur Anzeige eines in einem Hohlraum vorhandenen Druckes, insbesondere für Druckmessungen in medizinischen Einrichtungen, wie Vakuum-Saugvorrichtungen zum Absaugen von Körperflüssigkeiten mittels einer Skala, mit einem hohlen, flexiblen Anzeigeelement, dessen Innenraum mit dem Hohlraum verbunden ist, besteht das Anzeigeelement (4) aus einem an seinem freien Ende (12) verschlossenen spiralförmig angeordneten Rohr (5) aus biegsamen Material und daß im Rohr (5) eine Spiralfeder (6) angeordnet ist, die an ihren Enden mit dem Rohr (5) in Verbindung steht.

EP 0 381 123 A1

## Anzeigevorrichtung zur Anzeige eines in einem Hohlraum vorhandenen Druckes

Die Erfindung bezieht sich auf eine Anzeigevorrichtung nach dem Oberbegriff des Ansruches 1.

Eine Anzeigevorrichtung dieser Art ist in der DE-OS 35 23 843 beschrieben und dargestellt. Bei dieser bekannten Ausgestaltung wird die Anzeigevorrichtung durch ein Balgelement gebildet, dessen Innenraum mit dem Hohlraum in Verbindung steht und das sich mit veränderndem Druck im Hohlraum in seiner Längsrichtung ausdehnt und somit eine Anzeige für den Druck vorgibt.

Mit dieser bekannten Anzeigevorrichtung lassen sich Drücke nur ziemlich ungenau anzeigen, und zwar gilt dies insbesondere für niedrige Drücke bzw. für geringe Druckunterschiede. Es erscheint zwar möglich, ein Balgelement so auszugestalten, daß ein größerer Anzeigweg erreicht wird, jedoch läßt sich dies nur unter wesentlichen Einbußen der Linearität erreichen. Eine möglichst lineare Anzeige wird jedoch grundsätzlich angestrebt. Ein weiterer Nachteil dieser bekannten Ausgestaltung besteht in der verhälgnismäßig großen Bauweise, die durch die zylindrische Form des Balkelements vorgegeben ist.

Eine Anzeigevorrichtung mit linearer Druck-Anzeige ist in dem DE-GM 87 10 428 beschrieben und dargestellt. Bei dieser bekannten Ausgestaltung wird die Anzeigevorrichtung durch ein wenigstens teilweise transparentes starres Rohr gebildet, in dessen Innenraum eine unter dem Druck des Hohlraumes stehende Flüssigkeit auf- und abzusteigen vermag, wobei zur Erzielung der angestrebten linearen Anzeige der Querschnitt des Innenraumes sich in Richtung auf das geschlossene Rohrende verändert, insbesondere verjüngt. Diese bekannte Ausgestaltung hat sich in der Praxis als brauchbar erwiesen, jedoch ist sie schon wegen der Anordnung einer besonderen Steigflüssigkeit verhältnismäßig teuer in der Herstellung. Allerdings läßt sich ein verhältnismäßig großer Anzeigebereich erreichen.

Der Erfindung liegt die Aufgabe zugrunde, eine Anzeigevorrichtung der eingangs angegebenen Art so auszugestalten, daß bei Gewährleistung einer möglichst linearen Anzeige ein großer Anzeigeweg erreicht wird.

Diese Aufgabe wird durch die kennzeichnenden Merkmale des Anspruchs 1 gelöst.

Bei der erfindungsgemäßen Ausgestaltung führt bei einer Druckveränderung im Hohlraum das freie Ende des Anzeigeelements eine kreisbogenförmige Bewegung aus, wobei sich eine große lineare oder annähernd lineare Anzeigebewegung erreichen läßt. Die Größe der Anzeigebewegung und die Feinheit, mit der das Anzeigeelement sich auf Druckunterschiede im Hohlraum bewegt, sind abhängig von der Biegsamkeit des Anzeigeelements. Je größer die Biegsamkeit ist, umso größer ist auch die Reaktion des Anzeigeelements auf Druckunterschiede im Hohlraum. Bei einem leicht biegsamen Werkstoff für das Anzeigeelement läßt sich somit eine feine Ansprechbarkeit und eine deutliche Anzeige auch geringer Drücke bzw. Druckunterschiede erreichen. Anzustreben ist ein Werkstoff oder auch eine Form für das Anzeigeelement, der bzw. die zu einer möglichst gleichbleibenden Biegespannung im Anzeigeelement in den unterschiedlichen Bewegungsstellungen führt. Bei den Bewegungen des Anzeigeelements stellt sich die Spiralfeder aufgrund ihrer im Bewegungsbereich im wesentlichen gleichbleibenden Biegespannung eine Führungsgröße für die Bewegung dar. Eventuell im Anzeigeelement vorhandene nichtlineare Bewegungseigenschaften werden somit von der Linearität der Spiralfeder, die im Rahmen der Erfindung auch eine Blattfeder sein kann, weitgehend eliminiert.

Die Form der Spiralfeder gibt auch im wesentlichen die sich einstellende Form des Anzeigeelementes vor. Diese Form kann kreisförmig, spiralförmig oder auch wendelförmig sein. Bei einer spiral- oder wendelförmigen Ausgestaltung können auch mehrere Windungen oder Wendeln verwirklicht werden. Anzustreben ist, daß sich das freie Ende der Windung oder der Windungen bei deren Öffnen bei Druckanstieg und Schließen bei Druckabfall auf einem Kreisbogen bewegt, der gegenüber der Halterung des Anzeigeelementes im wesentlichen stationär ist, d. h., eine Kreisbewegung, deren Mittelpunkt unveränderlich ist.

Im Rahmen der Erfindung sind jedoch auch andere Bewegungsformen des freien Endes des Anzeigeelementes zur Anzeige des jeweiligen Druckes brauchbar. In jedem Fall ist bei einer Druckanzeige mittels Skala die Skala an die Bewegungsform des Anzeigeelementes anzupassen.

Bei einem Versuchsmodell mit mehreren wendelförmigen Windungen bzw. Wendeln zeigte sich eine geringförmige Vergrößerung des Durchmessers der Windungen bzw. Wendeln bei Druckanstieg und eine geringe Verringerung des Durchmessers bei Druckabfall. Gleichzeitig verlagerten sich die Windungen bzw. Wendeln bei Druckanstieg von der Halterung weg und bei Druckabfall zur Halterung hin, so daß bei der Benutzung eines zylindrischen Schlauches beim vorbeschriebenen Versuch die aus der Durchmesserveränderung resultierende Veränderung und die aus der Verlagerung resultierende Veränderung der Bewegung einander im wesentlichen ausgleichen, so daß sich eine im wesentlichen konstante bzw. stationäre Bewegungslinie für das freie Ende des Anzeigeelementes ergab.

Ein weiterer Vorteil der erfindungsgemäßen Ausgestaltung besteht darin, daß sie auf einer einfachen Bauweise beruht, die kostengünstig hergestellt werden kann, und zwar insbesondere dann, wenn ein dünnwandiger biegsamer Schlauch für das Anzeigeelement verwendet wird.

Die erfindungsgemäße Anzeigevorrichtung eignet sich insbesondere für Vakuum-Saugvorrichtungen oder sogenannte Saugflaschen zum Absaugen von Körperflüssigkeit, da sie eine genaue Anzeige bei niedrigen Drücken und Druckunterschieden ermöglicht. Dabei zeichnet sich die erfindungsgemäße Ausgestaltung auch durch eine handhabungsfreundliche Bauweise aus.

Vorteilhafte Weiterbildungen der Erfindung, die das Ansprechen der Anzeigevorrichtung auf Druckunterschiede, die Funktion und die Anzeige weiter verbessern sowie die Bauweise und Herstellung vereinfachen, sind in den Unteransprüchen beschrieben.

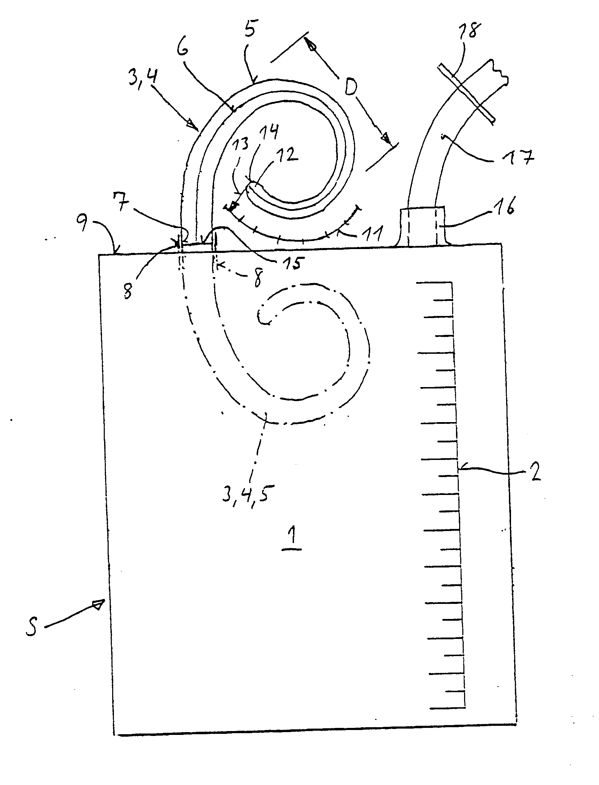

Nachfolgend wird die Erfindung anhand von in einer Zeichnung dargestellten bevorzugten Ausführungsbeispielen näher erläutert. In der Zeichnung ist eine Vakuum-Saugvorrichtung in Form einer sogenannten Saugflasche mit einer erfindungsgemäßen Anzeigevorrichtung in der Vorderansicht dargestellt.

Die allgemein mit S bezeichnete Saugvorrichtung bzw. Saugflasche besitzt eine in etwa quaderförmige Form und ist aus einem transparenten Kunststoffmaterial gefertigt, so daß die Menge der innerhalb einer Kammer 1 sich ansammelnden Sekretflüssigkeit anhand einer Skalenmarkierung 2 sehr leicht abgelesen werden kann, welche an der Vorderwandung der Saugflasche S angebracht sind.

Die allgemein mit 3 bezeichnete Anzeigevorrichtung besteht aus einem kreisbogen-, spiral- oder wendelförmig gebogenen Anzeigeelement 4, das durch ein Rohr 5 aus biegsamem Material, insbesondere dünner Wandstärke, und einer Spiralfeder 6 gebildet ist. Bei dickwandigeren Rohren 5 kann auch das Rohr selbst entsprechend der Spiralfeder 6 geformt sein. Beim vorliegenden Ausführungsbeispiel wird ein handelsüblicher leicht biegbarer Schlauch als Rohr 5 verwendet, dem die vorgegebene Form durch die Spiralfeder 6 aufgezwungen ist.

Das Rohr 5 ist an seinem Fußende 7, daß sich auch gerade erstrecken kann, in einem Anschlußstutzen 8 vorzugsweise an der Deckwand 9 dicht aufgenommen, z. B. verschraubt, verklebt oder ein- bzw. aufgepreßt, so daß sich der in der Saugflasche S vorhandene Druck in das Rohr 5 fortsetzen kann. Mit 11 ist eine Skala angedeutet, die in Verbindung mit einem am freien Ende 12 des Rohres 5 vorzugsweise quer angebrachten Zeiger 13 eine Anzeige des vorhandenen Druckes ermöglicht. Die Spiralfeder 6 ist vorzugsweise längs im Rohr 5 angeordnet, wobei sie sich vorteilhaft koaxial erstreckt, und zwar beim vorliegenden Ausführungsbeispiel vom Fußende 7 bis zum freien Ende 12 des Rohres 5, wobei sie innenseitig an der Stirnwand 14 des freien Endes 12 befestigt ist. An ihrem hinteren Ende ist die Spiralfeder 6 koaxial an einem Flanschteil 15 befestigt, dessen Form dem Querschnitt des Rohres 5 angepaßt ist und das mit seinem Umfang an der Wandung des Rohres 5 gehalten ist. Das Flanschteil 15 weist eine oder mehrere Ausnehmungen auf, so daß sich der in der Saugflasche S vorhandene Druck in das Rohr 5 fortpflanzen kann. Der Querschnitt des Rohres 5 ist beim vorliegenden Ausführungsbeispiel vorzugsweise rund.

Am oberen Ende weist die Saugflasche S bzw. der vorhandene Behälter einen Anschlußstutzen 16 auf, an welchem ein der Wunddrainage dienender Absaugschlauch 17 angeschlossen ist. Dieser Absaugschlauch 17 kann mit Hilfe einer Schlauchklemme 18, falls erforderlich, abgeklemmt werden. An der Deckwandung 9 ist schließlich noch eine nicht dargestellte Lasche mit einer entsprechenden Bohrung vorgesehen, an welcher die betreffende Saugflasche unter Einsatz von z. B. Kunststoffklemmelementen aufgehängt werden kann.

Die Größe des innerhalb der Kammer 1 herrschenden Unterdruckes kann mit Hilfe der Vakuum-Anzeigevorrichtung 3 abgelesen werden, deren Rohr-Windung oder Wendel mit zunehmendem Druck aufgeht und sich mit abnehmendem Druck um einen im wesentlichen konstanten Punkt, etwa dem Windungsmittelpunkt, aufwickelt. Dabei bewegt sich das freie Ende 12 des Rohres 5 auf einer etwa kreisbogenförmigen Linie entlang der Skala 11.

Zur Wunddrainage wird der Absaugschlauch 17 in die Wundhöhle eingebracht und die Schlauchklemme 48 geöffnet, so daß die innerhalb der Wundhöhle vorhandene Sekretflüssigkeit über den Ansaugschlauch 17 in das Innere der Saugflasche S geleitet wird. Der aktuelle Druck in der Saugflasche S bzw. in der Kammer 1 kann mittels der Vakuum-Anzeigevorrichtung 3 jederzeit abgelesen werden.

Im folgenden werden noch Maßangaben angeführt, die sich bei Versuchen als vorteilhaft gezeigt haben.

Der Durchmesser des Rohres 5 bzw. Schlauches beträgt etwa 2 bis 5 mm

Windungsdurchmesser D etwa 10 - 50 mm.

Die Anzahl der Windungen beträgt 1 - 5.

Die Querschnittsabmessung der Spiralfeder 6 beträgt etwa 1 -2 mm, vorzugsweise Rund- oder Rechteckprofil.

Öffnungswinkel (Skalenbericht) 180 - 360°.
Die Spiralfeder 6 besteht vorzugsweise aus Federstahl.

Im Rahmen der Erfindung ist es auch möglich, die Anzeigevorrichtung 3 im Hohlraum bzw. in der Kammer 1 der Saugflasche S anzuordnen, vorzugsweise spiegelbildlich an der Deckwand 9, wie es in der Zeichnung angedeutet ist.

Bei transparenten Saugflaschenwänden kann dabei die Skala 11 vorteilhaft an einer Seitenwand der Saugflasche S angebracht sein, so daß die Anzeige optisch zu erfassen ist. In einem solchen Fall ist es vorteilhaft, die Anzeigevorrichtung 3 nahe der Seitenwand anzuordnen, um Paralaxe beim Ablesen auszuschließen.

## Ansprüche

1. Anzeigevorrichtung zur Anzeige eines in einem Hohlraum vorhandenen Druckes, insbesondere für Druckmessungen in medizinischen Einrichtungen, wie Vakuum-Saugvorrichtungen zum Absaugen von Körperflüssigkeiten mittels einer Skala, mit einem hohlen, flexiblen Anzeigeelement, dessen Innenraum mit dem Hohlraum verbunden ist, **dadurch gekennzeichnet,** daß das Anzeigeelement (4) aus einem an seinem freien Ende (12) verschlossenen spiralfederförmig angeordneten Rohr (5) aus biegsamem Material besteht und daß im Rohr (5) eine Spiralfeder (6) angeordnet ist, die an ihren Enden mit dem Rohr (5) in Verbindung steht.

2. Anzeigevorrichtung nach Anspruch 1, **dadurch gekennzeichnet,** daß die Spiralfeder (6) sich koaxial im Rohr (5) erstreckt.

3. Anzeigevorrichtung nach Anspruch 1 oder 2, **dadurch gekennzeichnet,** daß das vordere Ende der Spiralfeder (6) an der Stirnwand (14) des Rohres (5) befestigt ist.

4. Anzeigevorrichtung nach wenigstens einem der Ansprüche 1 bis 3, **dadurch gekennzeichnet,** daß das hintere Ende der Spiralfeder (6) am Fußende des Rohres (5) befestigt ist.

5. Anzeigevorrichtung nach Anspruch 4, **dadurch gekennzeichnet,** daß das hintere Ende der Spiralfeder (6) an einem wenigstens eine Öffnung aufweisenden Flanschteil (15) befestigt ist, das an die Querschnittsform des Rohres (5) angepaßt und an der Innenwand des Rohres (5) gehalten ist.

6. Anzeigevorrichtung nach wenigstens einem der Ansprüche 1 bis 5, **dadurch gekennzeichnet,** daß das Rohr (5) durch einen Schlauch gebildet ist.

7. Anzeigevorrichtung nach wenigstens einem der Ansprüche 1 bis 6, **dadurch gekennzeichnet,** daß das Rohr (5) oder der Schlauch zum freien Ende (14) hin konvergieren.

8. Anzeigevorrichtung nach wenigstens einem der Ansprüche 1 bis 6, **dadurch gekennzeichnet,** das das Rohr (5) oder der Schlauch aus einer zylindrischen Ausgangsform gebogen ist.

9. Anzeigevorrichtung nach wenigstens einem der Ansprüche 1 bis 8, **dadurch gekennzeichnet,** daß das Rohr (5) oder der Schlauch aus Gummi oder Kunststoff besteht.

10. Anzeigevorrichtung nach wenigstens einem der Ansprüche 1 bis 9, **dadurch gekennzeichnet,** daß das Rohr (5) oder die Spiralfeder (6) mehr als eine Windung bzw. Wendel aufweist.

11. Anzeigevorrichtung nach Anspruch 10, **dadurch gekennzeichnet,** daß die Windungen koaxial nebeneinander liegen.

12. Anzeigevorrichtung nach wenigstens einem der Ansprüche 1 bis 11, **dadurch gekennzeichnet,** daß die Spiralfeder (6) aus Federstahl besteht.

13. Anzeigevorrichtung nach wenigstens einem der Ansprüche 1 bis 12, **dadurch gekennzeichnet,** daß die Spiralfeder (6) einen runden, quadratischen oder rechteckigen Querschnitt aufweist.

14. Anzeigevorrichtung nach wenigstens einem der Ansprüche 1 bis 13, **dadurch gekennzeichnet,** daß sie an den Hohlraum (1) der Saugvorrichtung (S), insbesondere zum Absaugen von Körperflüssigkeit angeschlossen ist.

15. Anzeigevorrichtung nach Anspruch 14, **dadurch gekennzeichnet,** daß das Anzeigeelement (4) an einem vorzugsweise an der Deckwand (9) der Saugvorrichtung (S) angeordneten Steck-oder Schraubanschluß (8) angeschlossen ist.

16. Anzeigevorrichtung nach Anspruch 15, **dadurch gekennzeichnet,** daß sie im Behälter der Saugvorrichtung (S) angeordnet ist.

17. Anzeigevorrichtung nach Anspruch 16, **dadurch gekennzeichnet,** daß die Wand des Behälters transparent ist, die Skala (11) an der Wand angebracht ist und die Anzeigevorrichtung (3) möglichst nahe hinter der Wand in sich parallel zur Wand erstreckender Position angeordnet ist.

**EUROPÄISCHER RECHERCHENBERICHT**

EP 90101753.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| X | <u>DE - C2 - 2 262 744</u> (SCHMAUS) * Fig. 4,6 * | 1,3-6, 9,12, 13 | G 01 D 5/42 G 01 L 7/04 |
| A | | 2,7,8, 10,11, 14-17 | |
| X | <u>US - A - 3 986 400</u> (SCHMAUS) * Fig. 1,2 * | 1,3-6, 8,9, 12,13 | |
| A | | 2,7, 10,11, 14-17 | |
| X | <u>US - A - 4 015 478</u> (SCHMAUS) * Fig. 1,2 * | 1,3-6, 8,9, 12,13 | |
| A | | 2,7, 10,11, 14-17 | |

----

RECHERCHIERTE
SACHGEBIETE (Int Cl⁵)

A 61 C 17/00
A 61 M 1/00
G 01 D 5/00
G 01 F 1/00
G 01 L 7/00

Der vorliegende Recherchenbericht wurde für alle Patentansprüche erstellt.

| Recherchenort | Abschlußdatum der Recherche | Prüfer |
|---|---|---|
| WIEN | 10-04-1990 | KUNZE |